# EUROPEAN PATENT APPLICATION

(11) **EP 3 922 202 A1**
(43) Date of publication of application: **15.12.2021**
(21) Application number: 20179793.3
(22) Date of filing: 12.06.2020
(51) Int. Cl.: A61B 18/20

(54) **SYSTEM AND METHOD FOR DETERMINING SKIN CONTACT FOR A PERSONAL CARE DEVICE**

(71) Applicant: Koninklijke Philips N.V., 5656 AG Eindhoven (NL)
(72) Inventor: Van den Elzen, Stefano Johannes, 5656 AE Eindhoven (NL); Kooijman, Gerben, 5656 AE Eindhoven (NL); Ismail, Rameez, 5656 AE Eindhoven (NL); Yuan, Zhaorui, 5656 AE Eindhoven (NL); Gebre, Binyam Gebrekidan, 5656 AE Eindhoven (NL)
(74) Representative: Philips Intellectual Property & Standards

(57) **Abstract**

According to an aspect, there is provided a system (40) configured to perform a personal care operation on skin of a subject. The system (40) comprises a processing unit (46) and a personal care device (2). The personal care device (2) comprises a housing (4) having an aperture (10) that is arranged in the housing (4) such that the aperture (10) is adjacent to the skin when the personal care device (2) is in contact with the skin and is to be used to perform the personal care operation on the skin. The personal care device (2) also comprises an imaging unit (44) disposed in the housing (4) and arranged to obtain images of the skin adjacent to the aperture (10) using light passing through the aperture (10) into the personal care device (2), wherein, in a skin contact detection mode of the system (40), a focal plane (66) of the imaging unit (44) is aligned with the aperture (10). The processing unit (46) is configured to receive, in the skin contact detection mode, one or more images obtained by the imaging unit (44) in the skin contact detection mode, and to process the one or more images to determine whether the personal care device (2) is in contact with the skin.

## Description

### FIELD OF THE INVENTION

This disclosure relates to personal care devices for performing a personal care operation on skin of a subject, and in particular to a system and method for determining whether a personal care device is in contact with skin.

### BACKGROUND OF THE INVENTION

Many personal care devices are to be used when a portion of the device is in contact with, or suitably close to, the skin of a subject. Examples include devices for the removal of unwanted hairs using various techniques such as shaving, electrolysis, plucking, laser and light therapies (known as photoepilation or Intense Pulsed Light, IPL) and injection of therapeutic anti-androgens. Personal care devices for hair growth reduction and treating acne also require contact with skin. Personal care devices can also be used for providing a massage to the subject, for providing physiotherapy, for applying patches to the subject (e.g. electrocardiogram electrodes, etc.) and for ultrasound measurements.

Light-based hair removal is a personal care operation used to inhibit the growth of hair by exposing the skin to bright flashes or pulses of light, which can be referred to as IPL (Intense Pulsed Light) where the light pulse is generated by a lamp or light bulb. Alternatively the flash or pulse can be generated using a laser or one or more light emitting diodes (LEDs). The light penetrates the skin and is absorbed - among other places - in the root of the hair. The temperature of the root of the hair will rise and subsequently the temperature of the surrounding tissue will also rise. The growth of the hair is inhibited if the temperature rise is sufficient. This process is known as photothermolysis.

Contact with skin is required for successful treatment and to prevent a light pulse being directed into other body parts such as eyes, which can result in injury. A personal care device can be prevented from flashing if no skin contact is detected. Thus, personal care devices can be provided with a skin contact sensor to measure a parameter that is indicative of whether the personal care device is in contact with skin. The parameter may be capacitance or contact pressure.

Similarly, light pulses may only be suitable (safe) for certain skin types, and the personal care device can be prevented from flashing if a non-safe skin tone is detected. A personal care device can be provided with a skin tone sensor (e.g. an optical sensor) to measure a parameter (such as melanin index) that is indicative of the tone of the skin that the personal care device is in contact with and is about to treat.

Currently, skin contact sensors and skin tone sensors are integral to the portion of the personal care device that is in contact with skin. However, it is often desirable for this portion of the device to be removable and interchangeable with different attachments, where each attachment may be suitable for a different application or body part. For example, in the case of a light-based personal care operation, different attachments can be provided for the main body parts (e.g. arm, leg, belly-bikini), the bikini line, the face and the armpits. At present, a skin contact sensor and skin tone sensor is required on all attachments.

### SUMMARY OF THE INVENTION

Imaging units (e.g. cameras) are increasingly being included in personal care devices to obtain images of areas of the subject that the personal care operation is to be performed on and that the personal care operation has already been performed on. The use of an imaging unit embedded in the personal care device can provide potential benefits such as treatment guidance via displacement measurement, and evaluating the effects of a personal care operation. To minimise the increase in cost of a personal care device due to the addition of an imaging unit and the associated processing circuitry, consideration is being given to whether the imaging unit can be used to perform the functions of some of the existing sensors in a personal care device, enabling those sensors to be omitted. In particular, it would beneficial to use the imaging unit for detecting contact with skin instead of requiring dedicated skin contact sensors (particularly where each separate attachment of the personal care device has respective skin contact sensor components).

Therefore it is an object to provide a system and method for determining whether a personal care device is in contact with skin using images obtained by an imaging unit.

According to a first specific aspect, there is provided a system configured to perform a personal care operation on skin of a subject. The system comprises a processing unit; and a personal care device. The personal care device comprises a housing having an aperture that is arranged in the housing such that the aperture is adjacent to the skin when the personal care device is in contact with the skin and is to be used to perform the personal care operation on the skin; and an imaging unit disposed in the housing and arranged to obtain images of the skin adjacent to the aperture using light passing through the aperture into the personal care device, wherein, in a skin contact detection mode of the system, a focal plane of the imaging unit is aligned with the aperture. The processing unit is configured to receive, in the skin contact detection mode, one or more images obtained by the imaging unit in the skin contact detection mode, and to process the one or more images to determine whether the personal care device is in contact with the skin.

In some embodiments, the imaging unit is configured such that the focal plane of the imaging unit is in a fixed position relative to the aperture. In alternative embodiments, the imaging unit is configured such that the focal plane of the imaging unit is variable, and the imaging unit is configured to adjust the focal plane to align with the aperture in the skin contact detection mode of the system.

In some embodiments, the processing unit is configured to process only part of a received image corresponding to one or more edges of the aperture.

In some embodiments, the aperture comprises an optically transparent aperture covering plate aligned with the aperture.

In some embodiments, the personal care device further comprises a first light source arranged within the personal care device to generate skin illuminating light when the imaging unit is to obtain the one or more images.

In some embodiments, the processing unit is configured to determine whether the personal care device is in contact with the skin based on a focusing quality and/or sharpness of the image. In these embodiments, the processing unit may be configured to analyse the one or more received images to determine values of parameters relating to focusing quality and/or sharpness, and to determine whether the personal care device is in contact with the skin based on the determined values of said parameters.

In alternative embodiments, the processing unit is configured to implement a machine learning, ML, model, that receives the one or more images as an input and determines whether the personal care device is in contact with the skin based on a classification of the one or more received images. In these embodiments, the ML model may be based on any of: a support vector machine, a decision tree, a random forest, an artificial neural network, a deep neural network or a convolutional neural network.

In some embodiments, the personal care device is for performing a light-based personal care operation. In these embodiments, the personal care device further comprises a second light source for generating treatment light to perform the light-based personal care operation.

In some embodiments, the personal care device is configured such that the personal care operation is performed on the skin through the aperture.

In some embodiments, the system is further configured to perform the personal care operation if the processing unit determines that the personal care device is in contact with the skin, and/or prevent the personal care operation from being performed if the processing unit determines that the personal care device is not in contact with the skin.

In alternative embodiments, the system is further configured to regulate power consumption of the personal care device based on whether the processing unit determines that the personal care device is in contact with the skin.

In some embodiments, the processing unit is separate from the personal care device. In alternative embodiments, the personal care device comprises the processing unit.

According to a second aspect, there is provided a method for determining whether a personal care device is in contact with skin. The method comprises receiving one or more images from an imaging unit in a personal care device, wherein the imaging unit is arranged to obtain the one or more images using light passing into the personal care device through an aperture in a housing of the personal care device, wherein, in a skin contact detection mode, a focal plane of the imaging unit is aligned with the aperture; and processing, in the skin contact detection mode, using a processing unit, the received one or more images to determine whether the personal care device is in contact with the skin.

In some embodiments, the imaging unit is configured such that the focal plane of the imaging unit is in a fixed position relative to the aperture. In alternative embodiments, the imaging unit is configured such that the focal plane of the imaging unit is variable, and the imaging unit is configured to adjust the focal plane to align with the aperture in the skin contact detection mode of the system.

In some embodiments, the step of processing comprises processing only part of a received image corresponding to one or more edges of the aperture.

In some embodiments, the aperture comprises an optically transparent aperture covering plate aligned with the aperture.

In some embodiments, the method further comprises generating skin illuminating light using a first light source arranged within the personal care device when the imaging unit is to obtain the one or more images.

In some embodiments, the step of processing comprises determining whether the personal care device is in contact with the skin based on a focusing quality and/or sharpness of the image. In these embodiments, the step of processing may comprise analysing the one or more received images to determine values of parameters relating to focusing quality and/or sharpness, and determining whether the personal care device is in contact with the skin based on the determined values of said parameters.

In alternative embodiments, the step of processing comprises inputting the received one or more images into a machine learning, ML, model, and the ML model determining whether the personal care device is in contact with the skin based on a classification of the one or more received images. In these embodiments, the ML model may be based on any of: a support vector machine, a decision tree, a random forest, an artificial neural network, a deep neural network or a convolutional neural network.

In some embodiments, the personal care device is for performing a light-based personal care operation. In these embodiments, the personal care device further comprises a second light source for generating treatment light to perform the light-based personal care operation.

In some embodiments, the personal care device is configured such that the personal care operation is performed on the skin through the aperture.

In some embodiments, the method further comprises performing the personal care operation if it is determined that the personal care device is in contact with the skin, and/or preventing the personal care operation from being performed if it is determined that the personal care device is not in contact with the skin.

In alternative embodiments, the method further comprises regulating power consumption of the personal care device based on whether it is determined that the personal care device is in contact with the skin.

In some embodiments, the processing unit is separate from the personal care device. In alternative embodiments, the personal care device comprises the processing unit.

According to a third aspect, there is provided a computer program product comprising a computer readable medium having computer readable code embodied therein, the computer readable code being configured such that, on execution by a suitable computer or processor, the computer or processor is caused to perform the method according to the second aspect or any embodiment thereof.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is an illustration of an exemplary personal care device according to an embodiment;
Fig. 2 is a block diagram of an exemplary system comprising a personal care device and an apparatus according to various embodiments;
Fig. 3 is a simplified cutaway view of the exemplary personal care device in Fig. 1;
Fig. 4(a) is a further cutaway view of view of part of the exemplary personal care device in Fig. 3 in contact with skin, and Fig. 4(b) is an exemplary image obtained by the imaging unit when the personal care device is in contact with skin;
Fig. 5(a) is a further cutaway view of view of part of the exemplary personal care device in Fig. 3 not in contact with skin, and Fig. 5(b) is an exemplary image obtained by the imaging unit when the personal care device care is not in contact with skin; and
Fig. 6 is a flow chart illustrating an exemplary method for determining whether a personal care device is in contact with skin.

### DETAILED DESCRIPTION OF THE EMBODIMENTS

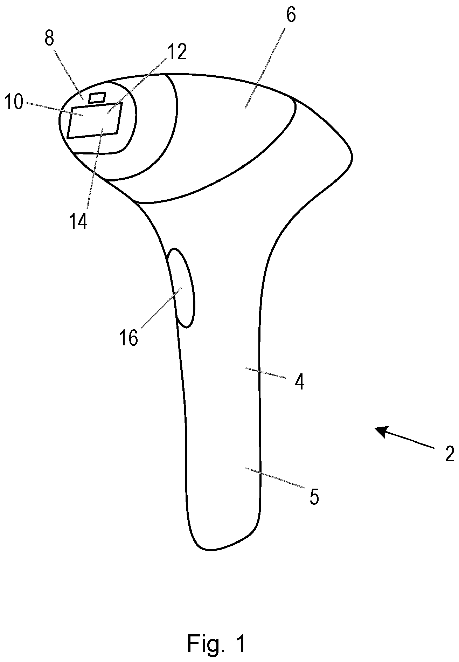

Fig. 1 is an illustration of an exemplary personal care device 2 according to an embodiment that can be used to apply an energy pulse (e.g. a light pulse) to an area of skin. It will be appreciated that the personal care device 2 in Fig. 1 is merely presented as an example of a personal care device 2 that the invention can be used with, and the personal care device 2 is not limited to the form shown in Fig. 1 or to being an energy-based personal care device. In some embodiments the personal care device 2 is to be held in one or both hands of a user during use. The personal care device 2 is for use on a body of a subject (e.g. a person or an animal) and is to perform some personal care operation on the body of the subject when the personal care device 2 is in contact with skin of the subject. In some embodiments, the personal care device 2 is to perform some personal care operation to the skin of the subject. Some exemplary personal care operations include, but are not limited to, the removal of unwanted hairs by any of shaving, electrolysis, laser and light therapies (known as photoepilation or Intense Pulsed Light, IPL); a dermatological (skin) treatment, including hair growth reduction, treating acne, a phototherapy treatment, skin rejuvenation, skin tightening, or port-wine stain treatment; and pain relief.

As described herein, the personal care device 2 is operated or used by a 'user', and the personal care device 2 is used on a body of a 'subject'. In some cases the user and the subject is the same person, i.e. the personal care device 2 is held in a hand and used by a user on themselves (e.g. used on the skin on their leg). In other cases the user and the subject are different people, e.g. the personal care device 2 is held in a hand and used by a user on someone else.

The personal care device 2 comprises a housing 4 that includes at least a handle portion 5 and a main body portion 6. The handle portion 5 is shaped to enable the user to hold the personal care device 2 with one hand. The main body portion 6 has a first end 8 that is to be placed into contact with skin of the sub when the personal care operation is to be performed on the body or skin of the subject.

In the embodiment illustrated in Fig. 1, the personal care device 2 is for performing a personal care operation using energy or energy pulses (e.g. light or light pulses). Thus, in Fig. 1 the first end 8 comprises an aperture 10 arranged in or on the housing 4 so that the aperture 10 can be placed adjacent to or on (i.e. in contact with) the skin of the subject. The personal care device 2 includes one or more energy sources 12 disposed inside the housing 4 that are for generating energy pulses that are to be applied to the skin of the subject via the aperture 10 and effect a personal care operation. The one or more energy sources 12 are arranged in the housing 4 so that the energy pulses are provided from the one or more energy sources 12 through the aperture 10. The aperture 10 may be in the form of an opening at the first end 8 of the housing 4. In some embodiments, the aperture 10 includes an aperture covering plate 14 that is transparent (e.g. optically transparent) to the energy pulses (i.e. the energy pulses can pass through the aperture covering plate 14).

In the exemplary embodiment shown in Fig. 1, the aperture 10 and aperture covering plate 14 (if present) have a generally rectangular shape, which results in a generally rectangular-shaped skin treatment region on the skin. It will be appreciated that the aperture 10 and/or aperture covering plate 14 can have any other desired shape. For example the aperture 10 and/or aperture covering plate 14 can be square, elliptical, circular, or any other polygonal shape.

The one or more energy sources 12 can generate any suitable type of energy for performing a personal care operation, for example light, sound, radio frequency (RF) signals, microwave radiation and plasma. In the case of an energy source 12 that generates light, the energy source 12 can be configured to generate a light pulse at any suitable or desired wavelength (or range of wavelengths) and/or intensities. For example, the energy source 12 can generate visible light, infra-red (IR) light and/or ultraviolet (UV) light. Each energy source 12 can comprise any suitable type of light source, such as one or more light emitting diodes (LEDs), a flash lamp (e.g. a Xenon flash lamp), a laser or lasers, etc. In a preferred embodiment, the personal care device 2 is for performing photoepilation, and the energy source(s) 12 are to provide intense light pulses. For example the energy source(s) 12 can provide light pulses with spectral content in the 560-1200 nanometre (nm) range for a duration of around 2.5 milliseconds (ms), as these wavelengths heat melanin in the hair and hair root by absorption, which puts the hair follicles in a resting phase, preventing hair regrowth. In the case of an energy source 12 that generates sound, the energy source 12 can be configured to generate a sound pulse at any suitable or desired wavelength (or range of wavelengths) and/or intensities. For example, the energy source 12 can be an ultrasound transducer.

The one or more energy sources 12 are configured to provide pulses of energy. That is, the energy source(s) 12 are configured to generate energy at a high intensity for a short duration (e.g. less than 1 second). The intensity of the energy pulse should be high enough to effect the personal care operation on the skin or body part adjacent the aperture 10.

The illustrated personal care device 2 also includes a user control 16 that can be operated by the user to activate the personal care device 2 so that the required personal care operation is performed on the body of the subject (e.g. the generation of an energy pulse by the one or more energy source(s) 12). The user control 16 may be in the form of a switch, a button, a touch pad, etc.

Although not shown in Fig. 1, the first end 8 can be formed as a removable attachment that is intended for use on particular body parts. The removable attachments are also referred to herein as removable head portions. A number of removable attachments can be provided that each have a respective shape and respective aperture size, and the attachment can be selected for use on the personal care device 2 based on the body part to be treated. For example different attachments can be provided for use on the face, for use in the armpits, for use at the bikini line, and for use generally on the body (e.g. the larger body surface areas). Each attachment has a respective aperture 10 and optical diffuser 14 in/on the aperture 10.

Although not shown in Fig. 1, the personal care device 2 may include a skin tone sensor that is positioned on or in the first end 8. The skin tone sensor can be used to determine a skin tone of the skin that the first end 8 is adjacent to or in contact with. The skin tone sensor may measure a parameter that is indicative of the skin tone of the skin, and generate a measurement signal that comprises a time-series of measurements of the parameter. Typically a skin tone sensor is used in a personal care device 2, particularly a photoepilator, to make sure that the light pulse has an intensity that is appropriate for the type of skin being treated, or even to prevent a light pulse being generated if the skin type is unsuitable for light pulses (e.g. darker skin which has a much higher melanin content).

A skin tone sensor can be a light sensor and the parameter measured by the light sensor can be an intensity or level of light at a particular wavelength or multiple wavelengths reflected from the skin. The measured intensity or level of reflected light at a particular wavelength(s) can be indicative of the skin tone. The measured intensity or level of reflected light can be based on the concentration of melanin in the skin, and thus the measured intensity or level can indicate the melanin concentration. The melanin concentration can be derived, for example, from measurements of light reflection at 660nm (red) and 880nm (infrared) wavelengths.

As noted above, conventional personal care devices can include dedicated sensors for detecting when or whether the personal care device is in contact with skin. In the case of a personal care device, particularly a photoepilator, the skin contact sensor is used to make sure that the personal care device is correctly in contact with skin before a light pulse is generated to avoid the light pulse being directed into the eyes of the user or subject. Typically these dedicated sensors can detect skin contact by measuring capacitance via a respective pair of electrical contacts or electrodes on the surface of the first end, with the measured capacitance being indicative of whether there is skin contact. Alternatively, these dedicated sensors can detect skin contact based on an intensity or level of light measured by a light sensor, or measure contact pressure via pressure sensors or mechanical switches. Since these dedicated skin contact sensors are typically integral to the portion of the personal care device that is in contact with skin and this portion of the device can be interchangeable with different attachments, a skin contact sensor is required on all attachments.

Thus, this disclosure provides a way to detect contact between a personal care device and the skin using images obtained by one or more imaging units provided within the housing of the personal care device, which means that separate skin contact sensors on the personal care device (and separate skin contact sensors on different attachments) are not required. Thus, as described further below, one or more imaging units are disposed in the housing 4 and is arranged to obtain images of skin adjacent to the aperture 10 using light passing through the aperture 10 (or possibly through respective apertures in the case of multiple imaging units) into the personal care device 2. An image or images obtained by the imaging unit(s) are provided to a processing unit, and the processing unit processes the image(s) to determine whether the personal care device 2 is in contact with skin. In the embodiments described below, the personal care device 2 comprises a single imaging unit that obtains images using light passing through the aperture 10 into the personal care device 2. However, in other embodiments the personal care device 2 can comprise multiple imaging units. These multiple imaging units can be arranged to image through respective parts of the aperture 10, for example respective parts around the edges of the aperture 10, or respective apertures can be provided in the housing 4 for each of the imaging units. In the case of multiple imaging units, each imaging unit may have a relatively narrow field of view so that the overlap in image content between the obtained images is reduced. Although the embodiments described below refer to a personal care device 2 that comprises a single imaging unit, those embodiments can be readily adapted to use multiple imaging units, and further details of the multiple imaging unit embodiments are not provided herein.

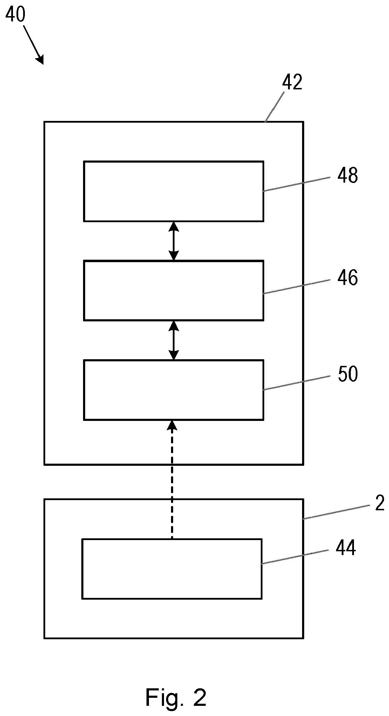

Fig. 2 is a block diagram of an exemplary system 40 comprising a personal care device 2 and an apparatus 42 for determining whether the personal care device 2 is in contact with skin. In Fig. 2 the apparatus 42 is a separate device to the personal care device 2, and thus the apparatus 42 may be in the form of an electronic device, such as a smart phone, smart watch, tablet, personal digital assistant (PDA), laptop, desktop computer, remote server, smart mirror, etc. In other embodiments, the apparatus 42, and particularly the functionality provided by the apparatus 42, is part of the personal care device 2. In yet other embodiments, the functionality of the apparatus 42 described below can be split between the personal care device 2 and a separate apparatus 42.

The personal care device 2 comprises an imaging unit 44 for obtaining images of skin adjacent to the aperture 10 using light passing through the aperture 10 into the personal care device 2. Fig. 2 only shows the imaging unit 44 in the personal care device 2, and it will be appreciated that in practice the personal care device 2 includes further components to those shown, for example the energy source(s) 12, a power source, a control unit, etc. In some embodiments, as described further below, one or more light sources may be provided that are associated with the imaging unit 44 and that are used to generate light when the imaging unit 44 is to obtain images. The light generated by these one or more light source(s) is also referred to herein as 'skin illuminating light', although it will be appreciated that the light will only be illuminating skin when skin is in contact with or close to the personal care device 2. This light source is also not shown in Fig. 2. The imaging unit 44 may include any suitable components for capturing an image, multiple images or a series of images (e.g. a video sequence), for example a charge-coupled device (CCD) and one or more lenses and/or mirrors. In some embodiments, the imaging unit 44 is a camera, such as a digital camera.

The apparatus 42 comprises a processing unit 46 that generally controls the operation of the apparatus 42 and enables the apparatus 42 to perform the method and techniques described herein. Briefly, the processing unit 46 receives one or more images from the imaging unit 44 and processes the image(s) to determine whether the personal care device 2 is in contact with skin.

Thus the processing unit 46 can be configured to receive the image(s) from the imaging unit 44, either directly in embodiments where the apparatus 42 is part of the personal care device 2, or via another component in embodiments where the personal care device 2 is separate from the apparatus 42. In either case, the processing unit 46 can include or comprise one or more input ports or wires for receiving the images (or signals carrying information representing the image(s)) from the imaging unit 44 or the other component as appropriate. The processing unit 46 can also include or comprise one or more output ports or wires for outputting a signal indicating whether the personal care device 2 is in contact with skin.

The processing unit 46 can be implemented in numerous ways, with software and/or hardware, to perform the various functions described herein. The processing unit 46 may comprise one or more microprocessors or digital signal processors (DSPs) that may be programmed using software or computer program code to perform the required functions and/or to control components of the processing unit 46 to effect the required functions. The processing unit 46 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor).

The processing unit 46 can comprise or be associated with a memory unit 48. The memory unit 48 can store data, information and/or signals (including image(s)) for use by the processing unit 46 in controlling the operation of the apparatus 42 and/or in executing or performing the methods described herein. In some implementations the memory unit 48 stores computer-readable code that can be executed by the processing unit 46 so that the processing unit 46 performs one or more functions, including the methods described herein. In particular embodiments, the program code can be in the form of an application for a smart phone, tablet, laptop, computer or server. The memory unit 48 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In the embodiment shown in Fig. 2, as the apparatus 42 is shown as being separate from the personal care device 2/imaging unit 44, the apparatus 42 also includes interface circuitry 50 to enable the apparatus 42 to receive the image(s) from the imaging unit 44. The interface circuitry 50 in the apparatus 42 enables a data connection to and/or data exchange with other devices, including any one or more of the imaging unit 44, the personal care device 2, servers, databases, user devices, and sensors. The connection to the imaging unit 44 (or any electronic device, such as treatment device 2) may be direct or indirect (e.g. via the Internet), and thus the interface circuitry 50 can enable a connection between the apparatus 42 and a network, or directly between the apparatus 42 and another device (such as imaging unit 44 and/or personal care device 2), via any desirable wired or wireless communication protocol. For example, the interface circuitry 50 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 50 (and thus apparatus 42) may include one or more suitable antennas for transmitting/receiving over a transmission medium (e.g. the air). Alternatively, in the case of a wireless connection, the interface circuitry 50 may include means (e.g. a connector or plug) to enable the interface circuitry 50 to be connected to one or more suitable antennas external to the apparatus 42 for transmitting/receiving over a transmission medium (e.g. the air). The interface circuitry 50 is connected to the processing unit 46.

Although not shown in Fig. 2, the apparatus 42 may comprise one or more user interface components that includes one or more components that enables a user of apparatus 42 to input information, data and/or commands into the apparatus 42, and/or enables the apparatus 42 to output information or data to the user of the apparatus 42. The user interface can comprise any suitable input component(s), including but not limited to a keyboard, keypad, one or more buttons, switches or dials, a mouse, a track pad, a touchscreen, a stylus, a camera, a microphone, etc., and the user interface can comprise any suitable output component(s), including but not limited to a display unit or display screen, one or more lights or light elements, one or more loudspeakers, a vibrating element, etc. In some embodiments the user interface component(s) can be used to provide an indication to the user as to whether the personal care device 2 is in contact with skin, or in correct contact with skin. This indication can be determined based on the result of the processing of the image(s) by the processing unit 46. In embodiments the indication can be provided as a visual indication, an audible indication and/or a tactile indication.

It will be appreciated that a practical implementation of an apparatus 42 may include additional components to those shown in Fig. 2. For example the apparatus 42 may also include a power supply, such as a battery, or components for enabling the apparatus 42 to be connected to a mains power supply.

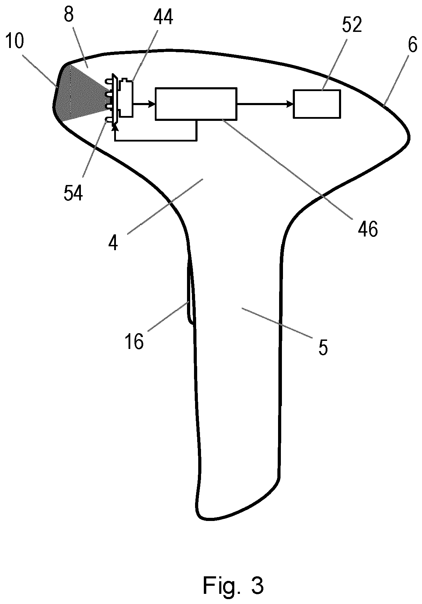

Fig. 3 is a simplified cutaway view of the exemplary personal care device 2 in Fig. 1, showing an arrangement of the imaging unit 44 in the personal care device 2. In this embodiment, the apparatus 42, and in particular the processing unit 46, is part of the personal care device 2, and is shown as being connected to the imaging unit 44. The imaging unit 44 is arranged in the housing 4 so that it is able to obtain images using light passing through the aperture 10 into the personal care device 2. A user interface component 52 connected to the processing unit 46 is also shown, and this user interface component 52 can be used to provide feedback to the user of the personal care device 2 based on the result of the processing of image(s) by the processing unit 46.

Fig. 3 also shows one or more light sources 54 that are associated with the imaging unit 44 and that are used to generate light when the imaging unit 44 is to obtain images. When the aperture 10 is positioned against the skin, it may be difficult for the imaging unit 44 to obtain a good image of the skin due to low light conditions. The light source(s) 54 are therefore arranged in the housing 4 so that the light generated by the light source(s) 54 can pass through the aperture 10 (and aperture covering plate 14, if present) to illuminate any object (including skin) that is close to, or in contact with, the aperture 10. The one or more light source(s) 54 can be LEDs, or any other suitable type of light source. The one or more light source(s) 54 can emit white light (or colours (e.g. RGB) that combine to provide white light), light of specific colours, light having specific spectral content, or infrared light. Illuminating the skin using light of different colours can enable the images obtained by the imaging unit 44 to include colour information so that they can be used to determine skin tone as well as skin contact. As another example, illuminating the skin using infrared light can enable a melanin index of the skin to be determined. Typically, the one or more light source(s) 54 are different to any light source(s) 12 that are provided for performing the personal care operation.

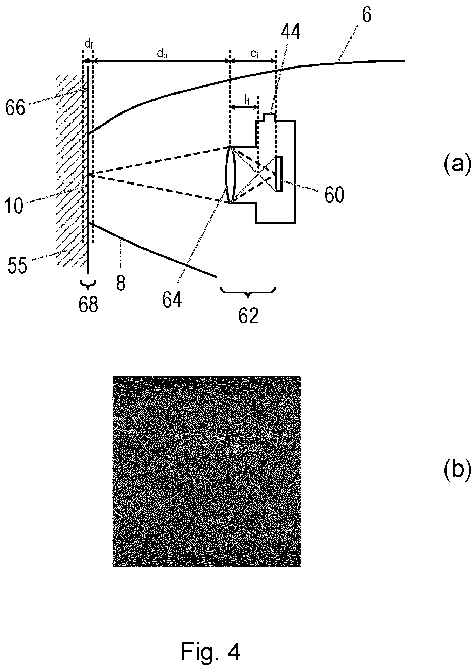

Fig. 4(a) is a further cutaway view of view of part of the exemplary personal care device in Fig. 3 when it is in contact with skin 55. Fig. 4(a) shows part of the main body portion 6 that includes the imaging unit 44. Two components of the imaging unit 44 are also shown, namely an image sensor 60 and an optical focusing arrangement 62, that in this embodiment comprises a single lens 64. The image sensor 60, for example a charge-coupled device (CCD), senses incident light and outputs an image signal (to the processing unit 46) comprising information on, or information representing, the incident light. The optical focusing arrangement 62 is present in front of the image sensor 60, and acts to focus incident light on to the image sensor 60. It will be appreciated that although only one lens 64 is shown in the optical focusing arrangement 62, a typical optical focusing arrangement 62 can include more than one lens 64.

According to the techniques described herein, skin contact detection is based on whether an image of skin obtained by the imaging unit 44 is in focus or sharp, or not. That is, the processing unit 46 can determine that the personal care device 2 is in contact with skin if an obtained image includes skin that is in focus, and the processing unit 46 can determine that the personal care device 2 is not in contact with skin (or not in proper contact with skin) if an obtained image does not include skin, or does not include skin that is in focus. To enable this, the imaging unit 44 (and particularly the optical focusing arrangement 62) is configured so that the focal plane 66 of the imaging unit 44 is aligned with the aperture 10, at least during a skin contact detection mode of the system 40 in which skin contact is to be detected. As appreciated by those skilled in the art, the focal plane of a lens 64, or more generally of an optical focusing arrangement 62, is a plane that is perpendicular to the optical axis of the lens 64/optical focusing arrangement 62 that coincides with the point of focus in front of the lens 64/optical focusing arrangement 62. In this way, when the personal care device 2 is in contact with skin 55 as shown in Fig. 4(a), the skin 55 will be at the aperture 10, and aligned with the focal plane 66, and an image of the skin obtained by the imaging unit 44 will be in focus. In Fig. 4(a) various distances are labelled: the image distance dᵢ (the distance between the lens 64 and the image sensor 60), the object distance dₒ (the distance between the lens 64 and the aperture 10), and the focal length l_{f} of the lens 64.

To improve the detection of the personal care device 2 not being in contact with skin 55, or not being in suitable contact with skin 55, the imaging unit 44 can be configured so that the depth of field of the imaging unit 44 is narrow or small. As appreciated by those skilled in the art, the depth of field of a lens 64/optical focusing arrangement 62 is the distance between the nearest and furthest points from the lens 64/optical focusing arrangement 62 in which an imaged object will be in focus. The depth of field is denoted df and is shown by dashed lines 68 in Fig. 4(a). The depth of field 68 is preferably sufficiently narrow or small that if the personal care device 2 is close to skin 55 but not in correct contact (i.e. correct for the purposes of performing the personal care operation), the skin 55 will not be in focus in an image obtained by the imaging unit 44. For example, a sufficiently narrow or small depth of field can be in the range of 2-3 millimetres (mm). Thus, if the skin 55 is more than 1-2 mm from the aperture 10 of the personal care device 2, then the skin 55 will not be fully in focus in an obtained image.

Fig. 4(b) is an exemplary image 70 obtained by the imaging unit 44 in the personal care device 2 when the personal care device 2 is in contact with skin 55. Elements of the skin in the image 70 are in focus and visible, such as various hairs on the skin.

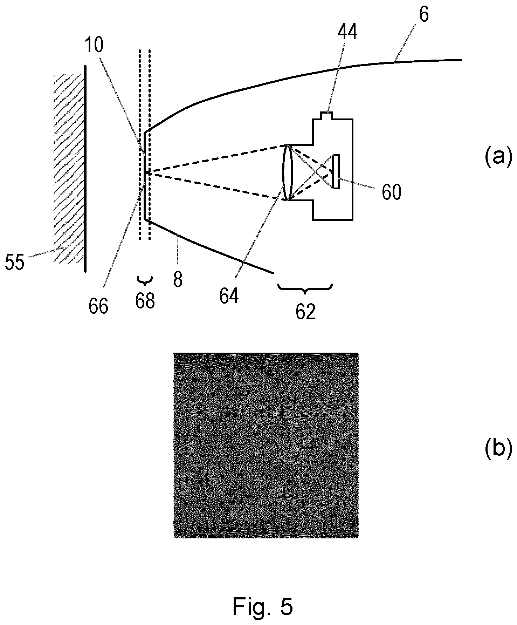

Fig. 5(a) shows the personal care device 2 from Fig. 4(a) that is spaced from the skin 55 by a small distance. At least during the skin contact detection mode of the system 40 in which skin contact is to be detected, the focal plane 66 of the imaging unit 44 is aligned with the aperture 10, and the skin 55 is outside of the imaging unit's depth of field 68, which means that an image of the skin 55 obtained when the personal care device 2 is in this position will not be in focus. Fig. 5(b) is an exemplary image 72 obtained by the imaging unit 44 in the personal care device 2 of the same area of skin 55 as in Fig. 4(b), when the personal care device 2 is spaced from the skin 55 as shown in Fig 5(a). In Fig. 5(b) it can be seen that elements of the skin in the image 70 are not in focus, or not clearly visible.

As noted above, the imaging unit 44 (and particularly the optical focusing arrangement 62) is configured so that the focal plane 66 of the imaging unit 44 is aligned with the aperture 10, at least during a skin contact detection mode in which skin contact is to be detected. In some embodiments, the focal plane 66 of the imaging unit 44/optical focusing arrangement 62 is in a fixed position relative to the aperture 10, and in particular the focal plane is fixed at the aperture 10. In other words, the focal plane remains aligned with the aperture 10, even when skin contact is not being detected (i.e. even when the system 40 is not operating in a skin contact detection mode).

However, in alternative embodiments, the imaging unit 44/optical focusing arrangement 62 can be configured so that the focus of the imaging unit 44/optical focusing arrangement 62 is variable or adjustable when the system 40 is not operating in the skin contact detection mode to enable focused images to be obtained of objects or subjects that are not in contact with the personal care device 2/aperture 10. This may be desirable where the images obtained by the imaging unit 44 can be used for other purposes in addition to skin contact detection, such as tracking movement of the personal care device 2 relative to a body part, or identifying where on the body the personal care device 2 is being used. In these embodiments, when skin contact is to be detected (i.e. the system 40 is operating in the skin contact detection mode), the focus of the imaging unit 44/optical focusing arrangement 62 is adjusted so that the focal plane is aligned with the aperture 10 to enable focused images to be obtained of skin when the personal care device 2 is in contact with skin.

In the embodiments shown in Figs. 1, 3, 4(a) and 5(a), the imaging unit 44 and the energy source(s) 12 share the same aperture 10 and aperture covering plate 14 (if present). That is, the imaging unit 44 generates images from light entering the personal care device 2 via the aperture 10, and the energy source(s) 12 emit energy out of the personal care device 2 through the aperture 10. However, in alternative embodiments, the personal care device 2 may be provided with a first aperture that may be used for detecting contact with skin (i.e. the imaging unit 44 can generate images from light entering the personal care device 2 via the first aperture) and a second aperture (e.g. the aperture 10) that may be used for the personal care operation (i.e. energy from the energy source(s) 12 passes out of the personal care device 2 through the second aperture).

Although the embodiments of the personal care device 2 illustrated in Figs. 1, 3, 4(a) and 5(a) are for performing a personal care operation using energy or energy pulses (e.g. light or light pulses), it will be appreciated that the personal care device 2 can be for performing other types of operations. For example, the personal care device 2 can be a shaver or hair clippers, in which case the main body portion 6 can comprise one or more cutting blades or foils for enabling hair to be cut when the main body portion 6 is in contact with skin. As another example, the personal care device 2 can be an ultrasound probe that is used to obtain ultrasound images. In this example, the main body portion 6 can include an ultrasound transducer for generating ultrasound waves, and an ultrasound receiver for receiving the ultrasound waves reflected back from the inside of the body. In these alternative types of personal care devices where the personal care operation is not performed via an aperture in the housing 4, the personal care device 2 is provided with aperture 10 at a location on the personal care device 2 that is in contact with skin when the personal care device 2 is in position to perform the personal care operation, so that the imaging unit is able to obtain images of skin via the aperture 10. This aperture 10 may include an aperture covering plate 12 as described above.

In embodiments where the personal care device 2 does not include an aperture covering plate 14, it is possible for the skin adjacent to the aperture 10 to 'dome' or 'bulge' when the personal care device 2 is in contact with skin. In particular, if the personal care device 2 is pressed on to the skin, the edge of the aperture 10 will depress the surface of the skin, and this can lead to the skin 'doming' or 'bulging' in the middle of the aperture 10 (i.e. the skin towards the middle of the aperture 10 extends a short way into the aperture 10). In this case, due to the alignment of the focal plane of the imaging unit 44 with the aperture 10, the processing unit 46 is configured to process parts of the image(s) that are around the edges of the aperture 10 in the image(s), as in these parts of the image(s) the skin will be in focus if the personal care device 2 is in contact with skin. Thus, in these embodiments, in detecting whether there is skin contact, the processing unit 46 can disregard central parts of the obtained image(s) where the skin may have 'domed' into the aperture 10 and may lie outside of the depth of field 68 of the imaging unit 44.

In embodiments where the aperture 10 includes an aperture covering plate 14, the plane of the aperture covering plate 14 is preferably aligned with the plane of the aperture 10. In this way the aperture covering plate 14 will prevent the skin adjacent to the aperture 10 from 'doming' into the aperture 10, and the processing unit 46 is able to analyse any or all parts of the image(s) obtained by the imaging unit 44 to determine if the personal care device 2 is in contact with skin.

In implementations where the personal care device 2 is for performing a personal care operation in which skin contact is required to ensure safety of the user and/or the subject, for example where the personal care device 2 is a photoepilator or other light-based personal care device, the personal care operation may be prevented from triggering or starting unless the processing unit 46 determines that the personal care device 2 is in contact with the skin.

In other implementations, skin contact detection can be used to regulate power consumption of the personal care device 2. In particular, if skin contact is detected, the personal care device 2 can be enabled to perform the personal care operation, and if skin contact is not detected, the personal care operation cannot be performed properly and the personal care device 2 can be deactivated or put into a lower power mode. For example, in the case of a personal care device 2 that includes a mechanical cutting element or mechanical shaving element for cutting or shaving hair, when skin contact is detected the cutting or shaving element can be activated so that hair can be cut, but when skin contact is not detected (or it is detected that the personal care device 2 is not in contact with skin), then the cutting or shaving element can be deactivated, or a cutting speed (e.g. motor speed) of the cutting or shaving element reduced.

As noted above, when it is to be determined whether the personal care device 2 is in contact with skin (e.g. at a time point when a personal care operation is to be performed), an image or images are obtained by the imaging unit 44 and are provided to the processing unit 46. The processing unit 46 processes the image(s) to determine whether the personal care device 2 is in contact with skin. In some embodiments, particularly where the skin contact detection has a safety aspect, the processing unit 46 may provide an indication of whether the personal care device 2 is in contact with skin for each received image.

In some embodiments, the processing unit 46 analyses the full obtained image to determine if there is skin contact, but in other embodiments the processing unit 46 may crop the obtained image or otherwise only analyse a part of the obtained image. This latter case may be used, for example, where the aperture 10 does not include an aperture covering plate 14, and the skin domes or bulges into the aperture 10 when the personal care device 2 is pressed on to skin, and so the processing unit 46 can crop the obtained image or otherwise only analyse the outer part of the image corresponding to the edges of the aperture 10 where the doming or bulging is smallest. On the other hand, if an aperture covering plate 14 is present, the processing unit 46 may crop the obtained image or otherwise only analyse the inner part of the image corresponding to the middle of the aperture 10 where the skin may be more uniformly illuminated by a light source associated with the imaging unit 44.

In some embodiments, the processing unit 46 can determine whether the personal care device 2 is in contact with skin based on whether the image is in focus (i.e. based on a focusing quality) and/or the image is sharp. The sharpness of an image relates to the definition of the edges of visible features in the image. For an image, the processing unit 46 can determine that there is no skin contact if the image is not in focus/blurred and/or not sharp, and the processing unit 46 can determine that there is skin contact if the image is in focus and/or is sharp. In these embodiments, the processing unit 46 can analyse the image to determine values of parameters relating to focusing quality (e.g. a measure of how focussed the image is) and/or sharpness (and optionally other image-related parameters, such as brightness, contrast, parameters relating to the presence of skin features in the image, etc.), and compare those values to one or more thresholds or ranges relating to skin contact/no skin contact to determine if there is skin contact. The processing unit 46 can output a signal indicating whether there is skin contact or not. In these embodiments, the processing unit 46 may apply conventional image processing techniques, for example as described in "Analysis of focus measure operators for shape-from-focus" by Pertuz, Said, Domenec Puig, and Miguel Angel Garcia, Pattern Recognition 46.5 (2013): 1415-1432.

In alternative embodiments, the processing unit 46 can use or implement a trained machine learning (ML) model to analyse the image(s) to determine if there is skin contact. Depending on the type of ML model used, the trained ML model can receive the image or images as input, analyse the image(s) and output a signal indicating whether there is skin contact or not. For other types of ML model, the processing unit 46 can determine values of parameters relating to focus/focusing quality and/or sharpness (and optionally other image-related parameters) and input these parameter values into the ML model. The ML model determines whether there is skin contact or not using the input parameter values, and outputs a signal indicating whether there is skin contact or not. Some types of ML model that can be used include a classical machine learning model such as feature extraction with support vector machines, decision trees, random forests, etc., or an artificial neural network, such as a deep neural network or a convolutional neural network, that has multiple layers between input and output layers and which identifies a linear or non-linear relationship between the input and output layers.

In some embodiments, the ML model is trained prior to deployment in the personal care device 2, for example using training data that includes skin images from a population of subjects. In this case the training data can include a number of images obtained when the personal care device 2 is at various distances/heights from skin of various different subjects (in which case the images will be blurred/out of focus), and a number of images when it is known that the personal care device 2 is in contact with different parts of the skin (of one or more different subjects), in which case the images will be in focus. Optionally some or all of the training data can be acquired in a controlled user test prior to deployment of the personal care device 2, or alternatively some or all of the training data can be acquired from users of the personal care device 2 that share their images and provide corresponding skin contact/no skin contact annotations for those images. Optionally the training data can also include images of other objects or backgrounds that be visible to the imaging unit 44 during use of the personal care device 2. The training data are used to train the ML model, for example using a supervised ML method. The training method results in a deployable trained classifier algorithm that outputs 'skin contact' or 'no skin contact' based on one or more input images.

In other embodiments the ML model can be trained during an initial calibration procedure performed on first use of the personal care device 2 by a user or subject. In this case, the user can be instructed to collect training data for the ML model by enabling the imaging unit 44 to obtain a number of images when it is known that the personal care device 2 is not in contact with skin, and a number of images when it is known that the personal care device 2 is in contact with different parts of the skin (of one or more different subjects). In further embodiments, both approaches can be used, i.e. the ML model can be trained prior to deployment, and a calibration procedure is performed so that the ML model is refined for the specific skin characteristics of the subject.

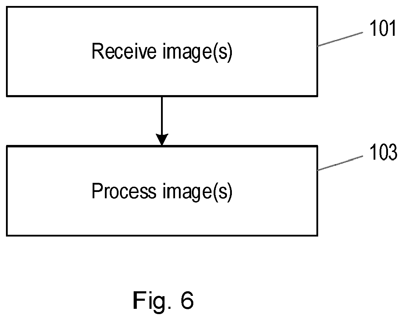

The flow chart in Fig. 6 illustrates an exemplary method according to the techniques described herein for determining whether the personal care device 2 is in contact with skin. One or more of the steps of the method can be performed by the system 40, such as the processing unit 46 in the apparatus 42, in conjunction with either of the memory unit 48 and interface circuitry 50 of the apparatus 42, and/or the imaging unit 44, as appropriate. The processing unit 46 may perform the one or more steps in response to executing computer program code that can be stored on a computer readable medium, such as, for example, the memory unit 48.

Although not shown in Fig. 6, an initial step of the method (when the system 40 is operating in the skin detection mode) can comprise the imaging unit 44 obtaining (generating) one or more images. The one or more images are obtained with the focal plane of the imaging unit 44 aligned with the aperture 10 of the personal care device 2.

In step 101, one or more images are received from the imaging unit 44. The image(s) can be received directly from imaging unit 44, for example in real-time or near real-time as the images are generated by the imaging unit 44. Alternatively the image(s) can be received from the imaging unit 44 in a separate personal care device 2 via the interface circuitry 50. As noted above, the imaging unit 44 is arranged to obtain the one or more images using light passing into the personal care device 2 through the aperture 10. At least when the system 40 is operating in a skin contact detection mode, a focal plane of the imaging unit 44 is aligned with the aperture 10. In the case of an imaging unit 44 where the focal plane is not adjustable, the focal plane of the imaging unit 44 will be aligned with the aperture 10 even when the system 40 is not operating in a skin contact detection mode. In the case of an imaging unit 44 where the focal plane is adjustable, the focal plane of the imaging unit 44 may still be aligned with the aperture 10 even when the system 40 is not operating in the skin contact detection mode.

In step 103, the one or more images are processed by the processing unit 46 (when the system 40 is operating in a skin contact detection mode) to determine whether the personal care device 2 is in contact with skin.

In some embodiments of step 103, due to the arrangement of the focal plane of the imaging unit 44 when the system 40 is operating in the skin contact detection mode, the processing unit 46 can process the image(s) with the aim of determining if the image is in focus and/or sharp. In some embodiments if the processing unit 46 determines that the image is not in focus or sharp, the processing unit 46 may determine that the personal care device 2 is not in contact with skin. In these embodiments if the processing unit 46 determines that the image is in focus and/or sharp, the processing unit 46 may determine that the personal care device 2 is in contact with skin.

In some embodiments of step 103, a trained ML model is used to process the one or more images to determine whether the personal care device 2 is in contact with the skin. The ML model can make an evaluation or classification for each image to determine whether the personal care device 2 is in contact with skin. In some embodiments the ML model directly receives the image(s) and performs all required analysis and processing of the images to determine whether there is contact between the personal care device 2 and skin. This is particularly the case for a ML model that is an artificial neural network, such as a deep neural network. In other embodiments, for example in the case of the use of a classical ML model, the image(s) can be processed before being provided to the ML model, for example to determine values for one or more features relating to the image, and these values can be provided to the ML model for analysis (optionally in addition to the image(s)) to determine whether the personal care device 2 is in contact with the skin.

Then, although not shown in Fig. 6, a signal can be output indicating whether the personal care device is in contact with skin. In some embodiments the indication can be a simple 'skin contact' or 'no skin contact'.

The signal may be provided to a user interface component of the apparatus 42 or personal care device 2 and the signal is configured to cause the user interface component to indicate whether the personal care device 2 is in contact with skin. For example, the signal could cause a red light on the personal care device 2 to be illuminated if it is determined that the personal care device 2 is not in contact with skin. Likewise the signal could cause a green light on the personal care device 2 to be illuminated if it is determined that the personal care device 2 is in contact with skin. The user of the personal care device 2 would be able to use these indications to determine whether to trigger the personal care operation at the current position of the personal care device 2. As another example, where the apparatus 42 is in the form of a smartphone or similar type of device, the feedback on whether the personal care device 2 is in contact with skin can be provided to the user or subject via an app (software application) executing on the apparatus 42. Those skilled in the art will be aware of other ways in which feedback on whether the personal care device 2 is contact with skin can be provided to a user, e.g. including using a display screen, a loudspeaker, haptic feedback, etc.

Alternatively (or in addition), where the personal care device 2 can automatically trigger a light pulse if the conditions are suitable (e.g. the personal care device 2 is in contact with skin, the tone of the skin the personal care device 2 is in contact with is suitable to receive a light pulse, etc.), the signal can be provided to a control unit of the personal care device 2, and the control unit can use the signal as part of taking the decision on whether to treat the area of skin currently adjacent to the aperture 10 with an energy pulse.

Therefore there is provided an improved system and method that enables contact between skin and a personal care device to be determined.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system (40) configured to perform a personal care operation on skin of a subject, the system (40) comprising:
a processing unit (46); and
a personal care device (2), comprising:
a housing (4) having an aperture (10) that is arranged in the housing (4) such that the aperture (10) is adjacent to the skin when the personal care device (2) is in contact with the skin and is to be used to perform the personal care operation on the skin;
an imaging unit (44) disposed in the housing (4) and arranged to obtain images of the skin adjacent to the aperture (10) using light passing through the aperture (10) into the personal care device (2), wherein, in a skin contact detection mode of the system (40), a focal plane (66) of the imaging unit (44) is aligned with the aperture (10); and
wherein the processing unit (46) is configured to receive, in the skin contact detection mode, one or more images obtained by the imaging unit (44) in the skin contact detection mode, and to process the one or more images to determine whether the personal care device (2) is in contact with the skin.

2. A system (40) as claimed in claim 1, wherein the imaging unit (44) is configured such that the focal plane (66) of the imaging unit (44) is in a fixed position relative to the aperture (10).

3. A system (40) as claimed in claim 1, wherein the imaging unit (44) is configured such that the focal plane (66) of the imaging unit (44) is variable, and the imaging unit (44) is configured to adjust the focal plane (66) to align with the aperture (10) in the skin contact detection mode of the system (40).

4. A system (40) as claimed in any of claims 1-3, wherein the processing unit (46) is configured to process only part of a received image corresponding to one or more edges of the aperture (10).

5. A system (40) as claimed in any of claims 1-4, wherein the aperture (10) comprises an optically transparent aperture covering plate (14) aligned with the aperture (10).

6. A system (40) as claimed in any of claims 1-5, wherein the personal care device (2) further comprises a first light source (54) arranged within the personal care device (2) to generate skin illuminating light when the imaging unit (44) is to obtain the one or more images.

7. A system (40) as claimed in any of claims 1-6, wherein the processing unit (46) is configured to determine whether the personal care device (2) is in contact with the skin based on a focusing quality and/or sharpness of the image.

8. A system (40) as claimed in claim 7, wherein the processing unit (46) is configured to analyse the one or more received images to determine values of parameters relating to focusing quality and/or sharpness, and to determine whether the personal care device (2) is in contact with the skin based on the determined values of said parameters.

9. A system (40) as claimed in any of claims 1-7, wherein the processing unit (46) is configured to implement a machine learning, ML, model, that receives the one or more images as an input and determines whether the personal care device (2) is in contact with the skin based on a classification of the one or more received images.

10. A system (40) as claimed in any of claims 1-9, wherein the personal care device (2) further comprises a second light source (12) for generating treatment light to perform the light-based personal care operation.

11. A system (40) as claimed in any of claims 1-10, wherein the personal care device (2) is configured such that the personal care operation is performed on the skin through the aperture (10).

12. A system (40) as claimed in any of claims 1-11, wherein the system (40) is further configured to perform the personal care operation if the processing unit (46) determines that the personal care device (2) is in contact with the skin, and/or prevent the personal care operation from being performed if the processing unit (46) determines that the personal care device (2) is not in contact with the skin.

13. A system (40) as claimed in any of claims 1-11, wherein the system (40) is further configured to regulate power consumption of the personal care device (2) based on whether the processing unit (46) determines that the personal care device (2) is in contact with the skin.

14. A method for determining whether a personal care device is in contact with skin, the method comprising:
- receiving (101) one or more images from an imaging unit in a personal care device, wherein the imaging unit is arranged to obtain the one or more images using light passing into the personal care device through an aperture in a housing of the personal care device, wherein, in a skin contact detection mode, a focal plane of the imaging unit is aligned with the aperture; and
- processing (103), in the skin contact detection mode, using a processing unit, the received one or more images to determine whether the personal care device is in contact with the skin.

15. A computer program product comprising a computer readable medium having computer readable code embodied therein, the computer readable code being configured such that, on execution by a suitable computer or processor, the computer or processor is caused to perform the method of claim 14.
